# FASCICULE DE BREVET EUROPEEN

(11) **EP 0 726 735 B1**
(45) Date de publication et mention de la délivrance du brevet: **16.04.1997**
(21) Numéro de dépôt: 95900189.2
(22) Date de dépôt: 04.11.1994
(51) Int. Cl.: A61B 17/58

(54) **SYSTEME D'ENCLOUAGE CENTROMEDULLAIRE**
MARKRAUMNAGELSYSTEM
CENTRO-MEDULLARY NAILING SYSTEM

(30) Priorité: 05.11.1993 FR 9313159
(43) Date de publication de la demande: 21.08.1996
(73) Titulaire: Sokolow, Constantin, F-78100 Saint-Germain-en-Laye (FR); Lepine, Jacques, F-75003 Paris (FR)
(72) Inventeur: Sokolow, Constantin, F-78100 Saint-Germain-en-Laye (FR); Lepine, Jacques, F-75003 Paris (FR)
(74) Mandataire: Lejet, Christian
(86) Numéro de dépôt international: FR9401276
(87) Numéro de publication internationale: WO9512358

(56) Documents cités:
- DE-A- 3 924 610
- FR-A- 2 656 212
- GB-A- 2 115 701
- US-A- 4 016 874
- US-A- 4 756 307
- US-A- 5 122 141

## Description

La présente invention concerne un système d'enclouage centromédullaire permettant d'assurer un traitement par ostéosynthèse d'une fracture diaphysaire, particulièrement pour un os long.

Tout traitement d'une fracture diaphysaire exige une très grande précision quant à la longueur finale de l'os, de son axe, etc...

Les méthodes chirurgicales s'opposent selon que l'ostéosythèse est à foyer ouvert, c'est à dire nécessitant l'abord du foyer de la fracture, ou que l'ostéosynthèse est à foyer fermé, c'est à dire ne nécessitant pas l'abord du foyer de la fracture.

Dans une ostéosynthèse interne à foyer ouvert, on utilise des plaques vissées permettant de compresser le foyer, notamment lorsque la fracture est spiroïde ou oblique longue. Parmi d'autres, les documents GB-A-2 115 701 et US-A-4 016 874 décrivent ainsi des systèmes à foyer ouvert et à vissage centromédullaire. Le document US-A-5 122 141 décrit un système à foyer ouvert, mais mettant en oeuvre un vissage centromédullaire accessible par l'épiphyse de l'os

Dans une ostéosynthèse interne à foyer fermé, l'implant est mis en place dans le canal médullaire par l'épiphyse de l'os à distance du foyer diaphysaire.

Selon une première méthode à foyer fermé, on utilise un embrochage centromédullaire effectué à l'aide de broches élastiques précintrées groupées en faisceau dans la diaphyse, puis divergentes en direction de l'épiphyse opposée. Ce type d'ostéosynthèse est, par exemple décrit dans DE-A-3 924 610 et dans FR-A-2 656 212.

Selon une deuxième méthode à foyer fermé, on effectue un enclouage centromédullaire au moyen d'un clou ou tuteur métallique présentant une certaine élasticité ou non. Le clou est ensuite verrouillé ou non en fonction de la fracture. Un tel clou est, par exemple décrit dans US-A-5 035 697 et dans EP-A-0 428 985.

La présente invention concerne uniquement et spécifiquement la dernière méthode précitée, c'est à dire une ostéosynthèse interne à foyer fermé et enclouage centromédullaire.

Un des problèmes qui se posent au praticien est de pouvoir disposer à tout moment d'un nombre de clous lui permettant d'assurer l'immobilisation de l'os fracturé dans tous les cas, quelles que soient ses dimensions (longueur et diamètre) qui varient d'un sujet à l'autre.

On rappellera, en effet, que, pour l'os du tibia, par exemple, auquel l'enclouage selon la présente invention est notamment destiné, le diamètre de la cavité centromédullaire varie de 7 à 16 mm environ, tandis que la longueur de l'os est comprise entre 220 et 420 mm environ. Les clous les plus utilisés sont ceux dont le diamètre est de 11, 12 et 13 mm, et dont la longueur est de 300, 315, 330, 345, 360 et 380 mm.

Dans les établissements possédant une structure chirurgicale, il n'est pas toujours possible de posséder un stock de clous dont les dimensions en longueur et en diamètre permettent de faire face à tous les cas pouvant se présenter, et ceci d'autant plus que chaque clou est très cher. Le document US-A-4 805 607 précise à ce sujet que les systèmes d'enclouage traditionnels nécessitent jusqu'à quatre-vingt quatre clous de types différents.

La présente invention a pour but d'obvier à cet inconvénient en procurant aux hôpitaux et cliniques la possibilité de procéder à un enclouage centromédullaire sans avoir à posséder un stock important de tels clous.

Bien que cherchant de résoudre un tout autre problème, celui de l'allongement d'un os, le document FR-A-2 632 514 propose un clou centromédullaire dont la longueur peut être modifiée lorsqu'il a déjà été installé dans le canal centromédullaire. Dans ce but, le clou comprend une mécanique interne spécifique qui le rend très coûteux. En outre, ce document ne résout pas le problème de l'adaptation du diamètre du clou à celui du canal centromédullaire.

Le document US-A-4 756 307 propose un dispositif d'enclouage constitué par une pluralité de segments fins essentiellement de la longueur du canal et reliés entre eux parallèlement par un bio-adhésif résorbable. Ce dispositif a pour but de réduire les efforts de torsion et de courbure dès que les segments se dissocient. Mais il ne permet pas d'obtenir un clou d'une rigidité suffisante.

La présente invention concerne donc un système d'enclouage pour un traitement par ostéosynthèse interne à foyer fermé, ce système étant conçu aux fins de réduire le stock de clous requis dans les services de chirurgie osseuse. Il est, bien sûr, tout particulièrement destiné à être utilisé pour les os longs, tels le tibia et le fémur. A cet effet, le système d'enclouage est composé de pièces dont la fixation entre elles est simple et rapide, ces pièces pouvant être réunies immédiatement et solidement les unes aux autres avant l'opération pour constituer un clou rigide monolithique permettant ainsi d'immobiliser l'os fracturé.

Selon l'invention, le système est constitué par au moins trois pièces unitaires, une pièce basse ou distale de dimensions normalisées, une pièce haute ou proximale de dimensions normalisées, et une pièce intermédiaire de longueur déterminée, intercalée entre les pièces basse et haute, et présentant un diamètre D choisi en fonction de l'os concerné. La longueur du clou peut être augmentée en rajoutant des rallonges aux dimensions normalisées.

Les différentes pièces du système peuvent être reliées entre elles par vissage ou par précontrainte. Dans le premier cas, un filetage et un taraudage sont prévus aux extrémités en regard de chaque pièce. Dans le deuxième cas, les pièces sont reliées par accrochage et insertion longitudinale de câbles de liaison les traversant.

L'invention sera mieux comprise, et d'autres buts, avantages et caractéristiques de celle-ci apparaîtront plus clairement à la lecture de la description qui suit de modes préférés de réalisation donnés à titre non limitatif et à laquelle deux planches de dessins sont annexées sur lesquelles :
La Figure 1 représente schématiquement et séparément les trois pièces minimales d'un système d'enclouage, pièces prévues pour être reliées par vissage ;
La Figure 2 représente schématiquement une rallonge permettant d'augmenter la longueur du système ;
La Figure 3 représente schématiquement le clou obtenu au moyen du système selon un premier mode de réalisation de l'invention ;
La Figure 4 représente schématiquement une pièce intermédiaire vu en perspective et vu de dessus selon un deuxième mode de réalisation de l'invention ;
La Figure 5 représente schématiquement le clou obtenu au moyen du système selon le deuxième mode de réalisation de l'invention ; et
La Figure 6 illustre schématiquement les câbles de fixation des pièces du clou selon le deuxième mode de réalisation de l'invention.

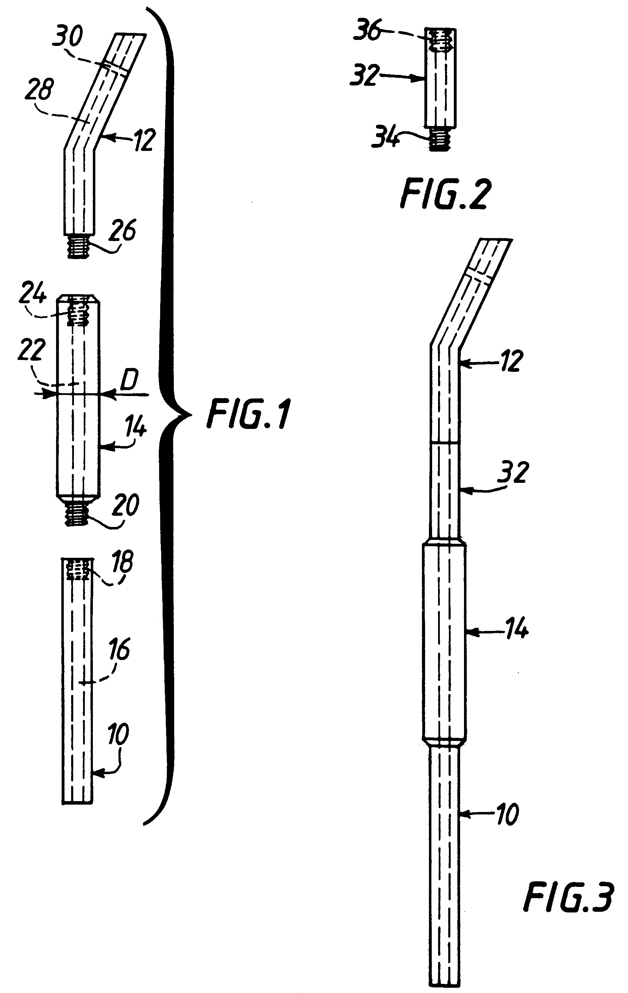

En référence maintenant aux Figures 1 à 3 relatives à un premier mode de réalisation d'un clou pour enclouage centromédullaire pour un traitement par ostéosynthèse interne à foyer fermé selon l'invention. En particulier, Figure 1, les trois pièces creuses élémentaires du système constitutif du clou ont été représentées séparément : une pièce normalisée basse ou distale 10, une pièce normalisée haute ou proximale 12, et une pièce intermédiaire 14 de longueur normalisée et dont le diamètre D est choisi en fonction du diamètre du canal centromédullaire.

La pièce normalisée basse 10 est percée d'un alésage central 16 qui se termine à son extrémité supérieure par un taraudage 18 dans lequel peut être introduite et vissée l'extrémité correspondante 20 en saillie et filetée de la pièce intermédiaire 14. Cette dernière présente également un alésage 22 dont l'extrémité supérieure 24 est taraudée de manière à coopérer avec l'extrémité filetée en saillie 26 de la pièce normalisée supérieure 12. Cette pièce supérieure est également alésée. Cet alésage est continu et identique pour les trois pièces de manière à ce que le praticien puisse y introduire un guide-clou pendant l'opération.
De façon plus générale, un taraudage est pratiqué dans la partie supérieure de chaque pièce en regard de la partie inférieure d'une autre pièce adjacente, laquelle partie inférieure présente une partie filetée faisant saillie et destinée à coopérer avec le taraudage précité.

L'assemblage des trois pièces est donc aisé et immédiat et ne comporte aucun risque de désassemblage. L'axe de la pièce supérieure 12 présente, dans sa partie haute 28, un angle de 25 º environ avec l'axe longitudinal des autres pièces, comme il est connu dans l'art. En outre, un orifice transversal 30 est prévu pour permettre l'extraction de l'ensemble lorsque la consolidation osseuse est considérée comme sensiblement achevée.

Figure 2, on a représenté une rallonge normalisée 32 comportant également un alésage axial dans le même but. L'extrémité supérieure de la rallonge présente un taraudage 36, tandis que son extrémité inférieure présente une partie filetée 34 faisant saillie axialement. Cette rallonge 32 permet simplement d'augmenter la longueur du système d'enclouage centromédullaire et d'adapter le clou final à la longueur de l'os. Cette rallonge 32 peut être indifférement disposée entre la pièce basse 10 et la pièce intermédiaire 14, ou entre la pièce intermédiaire 14 et la pièce haute 12. Bien évidemment, plusieurs rallonges identiques peuvent être utilisées ensemble pour obtenir la longueur voulue du clou.

Un clou final obtenu avec le système selon le premier mode de réalisation de la présente invention, a été représenté Figure 3. Le système d'enclouage comprend ici les quatre pièces qui viennent d'être décrites, c'est à dire la pièce basse 10, la pièce intermédiaire 14, une rallonge 32 et la pièce haute 12, reliées axialement ensemble par vissage.

Comme on peut le constater, en mettant en oeuvre l'invention, il n'est plus nécessaire, pour un centre de chirurgie osseuse, de posséder un jeu important de clous de tous les diamètres et de toutes les longueurs possibles pour faire face à tous les cas de fracture, puisque les pièces basses et hautes ont des dimensions normalisées, ainsi que les rallonges. Bien évidemment le diamètre de ces pièces doit être inférieur ou égal au diamètre minimal de la pièce intermédiaire. Seule la pièce intermédiaire doit être stockée en différents diamètres, par exemple en 7, 8, 9, 10, 11, 12, 13, 14 et 15 mm pour faire face à tous les cas. En outre, cette pièce intermédiaire 14 présente une longueur déterminée, ce qui permet de ne pas en multiplier le stockage.

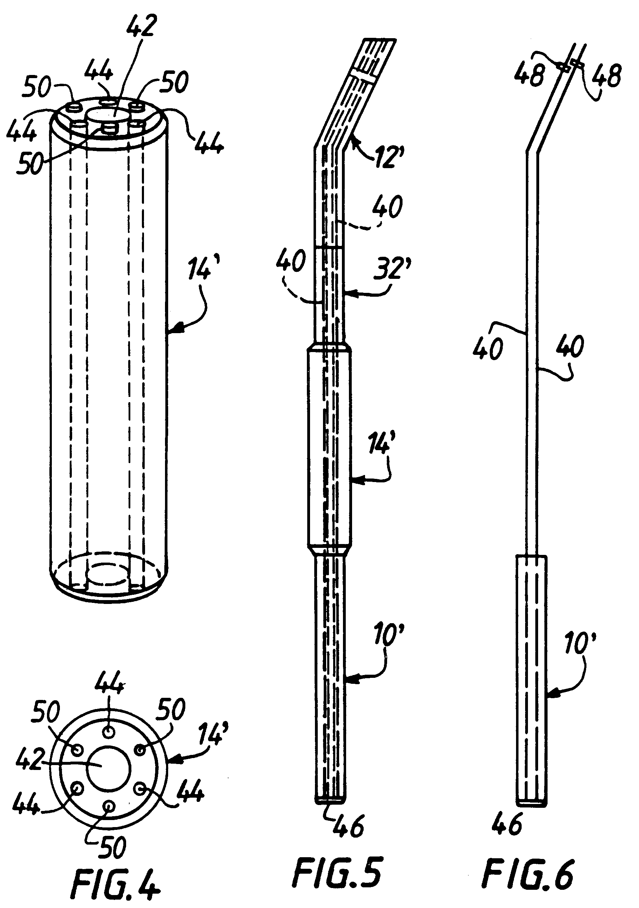

Les Figures 4 à 6 se rapportent à un deuxième mode de réalisation de l'invention, selon lequel l'assemblage des pièces est effectué par précontrainte au moyen de câbles de fixation 40 traversant de part en part les pièces en contact.

La Figure 4 représente isolément une pièce intermédiaire 14' vue en perspective et vue de dessus. Comme dans le premier mode de réalisation, cette pièce intermédiaire 14' doit exister en stock dans les neuf diamètres utilisables précités pour faire face à tous les cas.

Les pièces hautes 12' et basses 10', ainsi que les rallonges 32', ont la même structure générale que la pièce intermédiaire 14' représentée Figure 4. Elles présentent un alésage central 42 prévu pour l'insertion d'un guide-clou, et, sur leur pourtour, une pluralité d'alésages longitudinaux 44, de préférence trois, les traversant de part en part et prévus pour le passage des câbles de fixation 40.

Un clou complet ainsi réalisé selon le deuxième mode de réalisation du système d'enclouage selon l'invention a été représenté Figure 5 où il comprend quatre pièces comme le clou de la Figure 3.

Les câbles 40 sont bloqués dans la partie supérieure par des écrous 48 sur la pièce normalisée haute 12' et sont fixés sur une platine 46 maintenue à la partie inférieure de la pièce basse 10', comme représenté Figure 6.

Chacune des pièces peut, en outre, sur sa surface en regard d'une autre pièce, être pourvue d'accroches 50 contribuant à consolider, en coopération avec les câbles 40, la fixation instantanée et fiable des pièces entre elles. Ces accroches peuvent être favorablement constituées par une petite tige faisant saillie d'une surface supérieure d'une pièce et pénetrant dans un orifice correspondant pratiqué dans la surface inférieure de la pièce adjacente. En outre, comme représenté Figure 4, ces surfaces d'extrémité peuvent également être respectivement légèrement concaves et convexes pour favoriser l'assemblage des pièces et coopérer ensemble.

De la description qui précède, on peut aisément comprendre les avantages importants que procure la présente invention, du fait qu'elle permet de réduire dans une proportion non négligeable le nombre de clous classiques nécessaires pour répondre à tous les types d'enclouage, auxquels elle substitue un système d'enclouage centromédullaire composé de pièces de fabrication simple en série que le praticien peut relier instantanément et solidement entre elles avant de procéder à l'opération, du fait que les pièces constitutives des clous sont ainsi normalisées.

Bien que l'on ait représenté et décrit ce que l'on considère actuellement être les modes de réalisation préférés de la présente invention, il est évident que l'Homme de l'Art pourra y apporter différents changements et modifications sans sortir du cadre de la présente invention tel que défini par les revendications jointes.

## Revendications

1. Système d'enclouage centromédullaire pour un traitement par ostéosynthèse interne à foyer fermé, particulièrement destiné aux os longs, caractérisé en ce qu'il est constitué par au moins trois pièces unitaires, une pièce basse ou distale (10) de dimensions normalisées, une pièce haute ou proximale (12) de dimensions normalisées, et une pièce intermédiaire (14) de longueur donnée, intercalée entre les dites pièces basse et haute (10, 12), et présentant un diamètre D choisi en fonction de l'os concerné, les dites pièces (10, 12, 14) pouvant être réunies immédiatement et solidement les unes aux autres avant l'opération pour constituer un clou monolithique permettant d'immobiliser l'os fracturé.

2. Système d'enclouage selon la revendication 1 caractérisé en ce qu'il comprend, en outre, une rallonge (32) pouvant être insérée entre deux pièces et directement utilisable aux fins d'augmenter la longueur du clou.

3. Système d'enclouage selon la revendication 1 ou 2 caractérisé en ce que chaque pièce présente un alésage central (16, 22) destiné à accueillir un guide-clou.

4. Système d'enclouage selon l'une quelconque des revendications 1 à 3 caractérisé en ce que la fixation des différentes pièces entre elles est obtenue par vissage.

5. Système d'enclouage selon l'une quelconque des revendications 1 à 3 caractérisé en ce que la fixation des différentes pièces entre elles est obtenue par précontrainte.

6. Système d'enclouage selon la revendication 4 caractérisé en ce qu'un taraudage (18) est pratiqué dans la partie supérieure d'une pièce (10) en regard de la partie inférieure d'une autre pièce (14), laquelle partie inférieure présente une partie filetée (20) faisant saillie et destinée à coopérer avec le dit taraudage (18).

7. Système d'enclouage selon la revendication 5 caractérisé en ce que chaque pièce (10', 14', 12', 32') comporte sur son pourtour une pluralité d'alésages longitudinaux (44) permettant le passage des câbles (40) de fixation des pièces entre elles.

8. Système d'enclouage selon la revendication 7 caractérisé en ce que des accroches (50) sont prévues dans la partie supérieure d'une pièce (10') pour coopérer avec des accroches correspondantes prévue en regard dans la partie inférieure d'une pièce adjacente (14').

9. Système d'enclouage selon la revendication 7 ou 8 caractérisé en ce que la partie supérieure d'une pièce et la partie inférieure d'une pièce adjacente sont respectivement concave et convexe pour coopérer ensemble.

10. Système d'enclouage selon l'une quelconque des revendications 7 à 9 caractérisé en ce que les câbles de fixation (40) des différentes pièces sont bloqués dans la partie supérieure par des écrous (48) sur la dite pièce haute (12') et sont fixés sur une platine (46) maintenue à la partie inférieure de la dite pièce basse (10').

## Claims

1. Centro-medullary nailing system for a closed-site internal osteosynthesis treatment, particularly intended for the long bones, characterized in that it consists of at least three unitary components: a lower or distal component (10) of standardized dimensions, an upper or proximal component (12) of standardized dimensions, and an intermediate component (14) of given length interposed between the said upper and lower components (10, 12) and having a diameter D which is chosen as a function of the bone in question, it being possible for the said components (10, 12, 14) to be connected instantly and rigidly to one another before the operation so as to constitute a single-piece nail permitting immobilization of the fractured bone.

2. Nailing system according to Claim 1, characterized in that it additionally comprises an extension piece (32) which can be inserted between two components and which can be used directly for the purpose of increasing the length of the nail.

3. Nailing system according to Claim 1 or Claim 2, characterized in that each component has a central bore (16, 22) intended to receive a nail guide.

4. Nailing system according to any one of Claims 1 to 3, characterized in that the various components are secured to one another by screwing.

5. Nailing system according to any one of Claims 1 to 3, characterized in that the various components are secured to one another by prestressing.

6. Nailing system according to Claim 4, characterized in that an internal thread (18) is formed in the top part of one component (10) facing the bottom part of another component (14), which bottom part has a projecting externally threaded part (20) intended to cooperate with the said internal thread (18).

7. Nailing system according to Claim 5, characterized in that each component (10', 14', 12', 32') includes, on its circumference, a plurality of longitudinal bores (44) permitting the passage of the cables (40) for securing the components to one another.

8. Nailing system according to Claim 7, characterized in that catches (50) are provided in the top part of one component (10') in order to cooperate with corresponding catches provided facing them in the bottom part of an adjacent component (14').

9. Nailing system according to Claim 7 or Claim 8, characterized in that the top part of one component and the bottom part of an adjacent component are concave and convex, respectively, in order to cooperate.

10. Nailing system according to any one of Claims 7 to 9, characterized in that the cables (40) securing the various components are blocked in the top part by nuts (48) on the said upper component (12') and are secured on a plate (46) held in the bottom part of the said lower component (10').

## Patentansprüche

1. Markraumnagelsystem für eine Behandlung durch interne Osteosynthese bei geschlossenem Bruchherd bzw. geschlossener Bruchstelle, insbesondere bestimmt für die langen Knochen,
**dadurch gekennzeichnet,**
daß es gebildet wird durch wenigstens drei Einzelstücke: ein unteres oder distales bzw. fernes Stück (10) mit genormten Abmessungen, ein oberes oder proximales bzw. nahes Stück (12) mit genormten Abmessungen und ein zwischen das obere Stück (10 ) und das untere Stück (12) eingefügtes Zwischenstück (14) von bestimmter Länge und einem Durchmesser D, gewählt in Abhängigkeit von dem betreffenden Knochen, wobei die genannten Stücke (10, 12, 14) vor der Operation direkt und fest miteinander verbunden werden können, um einen monolithischen Nagel zu bilden, der ermöglicht, den gebrochenen Knochen zu immobilisieren.

2. Nagelsystem nach Anspruch 1, dadurch gekennzeichnet, daß es außerdem eine Verlängerung (32) umfaßt, die eingesetzt werden kann zwischen zwei Stücke und dazu benutzt wird, die Länge des Nagels zu vergrößern.

3. Nagelsystem nach Anspruch 1 oder 2, dadurch gekennzeichnet, daß jedes Stück eine zentrale Bohrung (16, 22) aufweist, dazu bestimmt, eine Nagelführung bzw. einen Nagelhaltor aufzunehmen.

4. Nagelsystem nach einem dar Ansprüche 1 bis 3, dadurch gekennzeichnet, daß die Befestigung der verschiedenen Stücke aneinander durch Verschraubung hergestellt wird.

5. Nagelsystem nach einem der Ansprüche 1 bis 3, dadurch gekennzeichnet, daß die Befestigung der verschiedenen Stücke aneinander durch Vorspannung hergestellt wird.

6. Nagelsystem nach Anspruch 4, dadurch gekennzeichnet, daß ein Innengewinde (18) vorgesehen ist im Oberteil eines Stücks (10), dem Unterteil eines anderen Stücks (14) gegenüberstehend, wobei dieser Unterteil einen gewindeten Teil (20) aufweist, vorstehend und dazu bestimmt, mit besagtem Innengewinde (18) zusammenzuwirken.

7. Nagelsystem nach Anspruch 5, dadurch gekennzeichnet, daß jedes Stück (10', 14', 12', 32') an seinem Umfang eine Vielzahl von Längsbohrungen (44) für den Durchgang von Kabeln (40) umfassen, die ermöglichen, die Stücke aneinander festzumachen.

8. Nagelsystem nach Anspruch 7, dadurch gekennzeichnet, daß Kupplungseinrichtungen (50) im bwz. am Oberteil eines Stücks (10') Vorgesehen sind, um zusammenzuwirken mit entsprechenden Kupplungseinrichtungen, die gegenüberstehend im Unterteil eines anstoßenden Stücks (14') vorgesehen sind.

9. Nagelsystem nach Anspruch 7 oder 8, dadurch gekennzeichnet, daß der Oberteil eines Stücks und der Unterteil eines anstoßenden Stücks zum Zusammenwirken jeweils eine Vertiefung beziehungsweise einen Vorsprung aufweisen.

10. Nagelsystem nach einem der Ansprüche 7 bis 9, dadurch gekennzeichnet, daß die Befestigungskabel (40) der verschiedenen Stücke im Oberteil des oberen Stücks (12') durch Schrauben (48) blockiert werden und an einer Platine (46) befestigt werden, festgehalten am Unterteil des unteren Stücks (10').
